# EUROPEAN PATENT APPLICATION

(11) **EP 2 669 381 A1**
(43) Date of publication of application: **04.12.2013**
(21) Application number: 12004148.8
(22) Date of filing: 30.05.2012
(51) Int. Cl.: C12N 15/86

(54) **Method for expression of heterologous proteins using a recombinant negative-strand RNA virus vector comprising a mutated P protein**

(71) Applicant: AmVac AG, 6300 Zug (CH)
(72) Inventor: Wiegand, Marian, Dr., 81667 München (DE)
(74) Representative: Ricker, Mathias

(57) **Abstract**

The present invention provides a method of expressing at least one heterologous nucleic acid sequence in a cell, the method comprising introducing at least one heterologous nucleic acid sequence into a cell by infecting said cell with a recombinant negative-strand RNA virus vector comprising said at least one heterologous nucleic acid sequence, wherein the recombinant negative-strand RNA virus vector includes a viral genome coding for a mutated P protein, which leads to a loss of the viral genome replication ability without a loss of the viral transcription ability, and wherein said at least one heterologous nucleic acid sequence encodes a cellular reprogramming or programming factor or a therapeutic protein. In addition, the present invention provides a cell or a population of cells prepared *in vitro* by said method as well as a pharmaceutical composition comprising said cell or population of cells.

SEQUENCE LISTING

<110>  AmVac AG

<120>  Method for expression of heterologous proteins using a
       recombinant negative-strand RNA virus vector

<130>  108695P546EP

<160>  6

<170>  PatentIn version 3.5

<210>  1
<211>  18
<212>  DNA
<213>  Artificial Sequence

<220>
<223>  Forward primer beta actin

<400>  1
caagagatgg ccactgcc                                                    18


<210>  2
<211>  22
<212>  DNA
<213>  Artificial Sequence

<220>
<223>  Reverse primer beta actin

<400>  2
cttgatcttc atggtgctag ga                                               22


<210>  3
<211>  22
<212>  DNA
<213>  Artificial Sequence

<220>
<223>  Forward primer Nanog

<400>  3
ggacaggttt cagaagcaga ag                                               22


<210>  4
<211>  22
<212>  DNA
<213>  Artificial Sequence

<220>
<223>  Reverse primer Nanog

<400>  4
accattgcta gtcttcaacc ac                                               22


<210>  5
<211>  20
<212>  DNA
<213>  Artificial Sequence

<220>
<223>  Forward primer Oct4

<400>  5
atcctccctt tatccagccc                                                   20


<210>  6
<211>  20
<212>  DNA
<213>  Artificial Sequence

<220>
<223>  Reverse primer Oct4

<400>  6
agaaggcgaa gtctgaagcc                                                   20

## Description

### FIELD OF THE INVENTION

The present invention relates to a method of expressing at least one heterologous nucleic acid sequence in a cell using a recombinant negative-strand RNA virus vector which is replication-deficient but transcription-competent. In particular, the present invention relates to an *in vitro* method of reprogramming a cell to a less-differentiated state and *in vivo* and *in vitro* gene therapy methods using said recombinant negative-strand RNA virus vector. In addition, the present invention concerns a cell or a population of cells prepared by said *in vitro* methods and the use of the prepared cell or population of cells as a medicament.

### BACKGROUND OF THE INVENTION

Recombinant viral vectors are among several known agents available for the introduction of foreign genes into mammalian cells mediating stable transgenic expression. Different viruses have been used for this purpose including retroviruses, herpes viruses, adenoviruses, and adeno-associated viruses. These recombinant viruses are particularly known for use in the production of viral vaccines or as gene therapy vectors for the treatment of gene deficiency disorders. Other applications of recombinant viruses include the de-differentiation of cells to pluripotent cells known as induced pluripotent stem (iPS) cells by retrovirus- or lentivirus-mediated transduction and expression of specific cellular re-differentiating or reprogramming factors.

A serious problem associated with the delivery of genes in the above-described virus-mediated approaches is the integration of genetic material from a viral vector into the host cell genomic DNA which may cause malignant transformation of the host cell. In particular, retrovirus-mediated delivery of genes encoding, for example, reprogramming factors, can result in genomic integration of the transgene. This may trigger activation of oncogenes or disrupt tumor suppressor genes, leading to malignant cell transformation. Another problem associated with integrating viral vectors is the fact that integrated and down-regulated transgenes may be re-activated to cause cellular transformation.

Other concerns include the possibility that the virus used for gene delivery may be transmitted to neighbouring healthy cells of the patient or from the patient to other individuals or into the environment. A further problem is that the virus vector may persist and cause adverse effects, such as an immune reaction against viral components or delayed effects of viral infection. Moreover, the prolonged expression of foreign genes may also result in an autoimmune-like reaction to self antigens or interfere with cellular processes like signalling pathways.

In order to increase the safety of delivery of foreign genes to a host cell in ex *vivo* and *in vivo* procedures, in particular with a view to the undesirable spread of the viral vectors, different strategies have been proposed. For example, in WO 2006/084746 A1 there is described a replication-deficient, but transcription-competent, negative-strand RNA virus. Due to its improved safety profile, this virus is described to be suitable for use as a live vaccine.

In addition, to avoid any problems caused by gene insertion into the genome of a target cell, it was attempted to introduce viral genetic constructs that do not integrate in the target cell's genome and allow the transient expression of a heterologous gene product. For example, WO 2010/008054 A1 describes a method for the production of an ES (embryonic cell)-like cell using a chromosomally non-integrating viral vector such as a Sendai virus vector. However, this viral vector is able to efficiently replicate in the infected target cell and, thus, the generated viral particles will be evenly distributed among daughter cells after each cell division. Thus, the ES-like cells produced contain undesirably high virus loads in their cytoplasm.

A similar approach is described in WO 2010/134526 A1 where a Sendai virus vector is used for reprogramming somatic cells into induced pluripotent stem (iPS) cells. The Sendai virus vector does not result in the integration of vector sequences into the host cell's genome and, thus, reduces the risk of tumorigenic transformation caused by the random integration of vector sequences into the host genome. However, since the Sendai virus vector described in WO 2010/134526 A1 is replication-competent, it needs to be removed with considerable effort after the reprogramming to ensure an adequate safety profile. Furthermore, as neither the delivery rate of the siRNAs, which are used in WO 2010/134526 A1 for the removal of the viruses, nor the siRNA-mediated expression inhibition will reach 100%, this approach cannot sufficiently obviate the health risks associated with the use of live viruses.

### OBJECTS OF THE INVENTION

In view of the prior art, it is the object of the present invention to provide a novel viral vector exhibiting an improved safety profile and being capable of efficiently expressing a heterologous nucleic acid sequence for a sufficiently long period period. In particular, the present invention aims to provide novel methods for delivering and expressing genes coding for therapeutic proteins in target cells as a gene therapy approach for various diseases. Furthermore, the present invention aims to deliver and express genes coding for cellular reprogramming or programming factors to reprogram an at least partially differentiated cell to a less differentiated cell that is suited for use in regeneration therapy or to program cells to adopt a desired differentiated state.

### SUMMARY OF THE INVENTION

In a first aspect, the present invention relates to a method of expressing at least one heterologous nucleic acid sequence in a cell. The method may be an *in vitro* or *in vivo* method and comprises the step of introducing at least one heterologous nucleic acid sequence into a cell by infecting said cell with a recombinant negative-strand RNA virus vector comprising said at least one heterologous nucleic acid sequence, wherein the recombinant negative-strand RNA virus vector includes a viral genome coding for a mutated P protein, which leads to a loss of the viral genome replication ability without a loss of the viral transcription ability, and wherein said at least one heterologous nucleic acid sequence encodes a cellular reprogramming or programming factor or a therapeutic protein.

In a preferred embodiment, an at least partially differentiated cell is reprogrammed to a less differentiated cell by an *in vitro* method comprising: (a) providing an at least partially differentiated cell from a donor, (b) introducing at least one heterologous nucleic acid sequence into said cell by infecting the cell with a recombinant negative-strand RNA virus vector comprising the at least one heterologous nucleic acid sequence, and (c) culturing the infected cell under conditions effective to express said at least one heterologous nucleic acid sequence, wherein the recombinant negative-strand RNA virus vector includes a viral genome coding for a mutated P protein, which leads to a loss of the viral genome replication ability without a loss of the viral transcription ability, and wherein said at least one heterologous nucleic acid sequence encodes a cellular reprogramming factor.

In another preferred embodiment, a cell to be programmed is programmed to a desired differentiated state by an *in vitro* method comprising: (a) providing a cell to be programmed from a donor, (b) introducing at least one heterologous nucleic acid sequence into said cell by infecting the cell with a recombinant negative-strand RNA virus vector comprising said at least one heterologous nucleic acid sequence, and (c) culturing the infected cell under conditions effective to express said at least one heterologous nucleic acid sequence, wherein the recombinant negative-strand RNA virus vector includes a viral genome coding for a mutated P protein, which leads to a loss of the viral genome replication ability without a loss of the viral transcription ability, and wherein said at least one heterologous nucleic acid sequence encodes a cellular programming factor.

In still another preferred embodiment, a genetic disorder is treated by an *in vivo* method comprising administering to a patient a recombinant negative-strand RNA virus vector comprising at least one heterologous nucleic acid sequence to introduce said at least one heterologous nucleic acid sequence into a cell of the patient, wherein the recombinant negative-strand RNA virus includes a viral genome coding for a mutated P protein, which leads to a loss of the viral genome replication ability without a loss of the viral transcription ability, and wherein said at least one heterologous nucleic acid sequence encodes a therapeutic protein capable of treating the genetic disorder.

In yet a further preferred embodiment, a genetic disorder is treated by an *in vitro* method comprising: (a) providing a cell from a donor, (b) introducing at least one heterologous nucleic acid sequence into said cell by infecting the cell with a recombinant negative-strand RNA virus vector comprising said at least one heterologous nucleic acid sequence, and (c) culturing the infected cell under conditions effective to express said at least one heterologous nucleic acid sequence and/or to allow the cell to expand, wherein the recombinant negative-strand RNA virus vector includes a viral genome coding for a mutated P protein, which leads to a loss of the viral genome replication ability without a loss of the viral transcription ability, and wherein said at least one heterologous nucleic acid sequence encodes a therapeutic protein capable of treating said genetic disorder.

In a second aspect, the present invention relates to a cell or a population of cells prepared by the *in vitro* methods of the present invention. This cell or population of cells may be used as a medicament, in particular as a medicament for use in gene therapy or regeneration therapy.

In a third aspect, the present invention relates to a pharmaceutical composition comprising a cell or a population of cells prepared by the *in vitro* methods of the present invention, or a redifferentiated cell or a population of redifferentiated cells derived from a cell or a population of cells prepared by the *in vitro* method of reprogramming of the present invention.

Preferred embodiments of the present invention are set forth in the appended claims.

The present invention may be understood more fully by reference to the following detailed description, the examples, and the accompanying drawings.

### BRIEF DESCRIPTION OF THE DRAWINGS

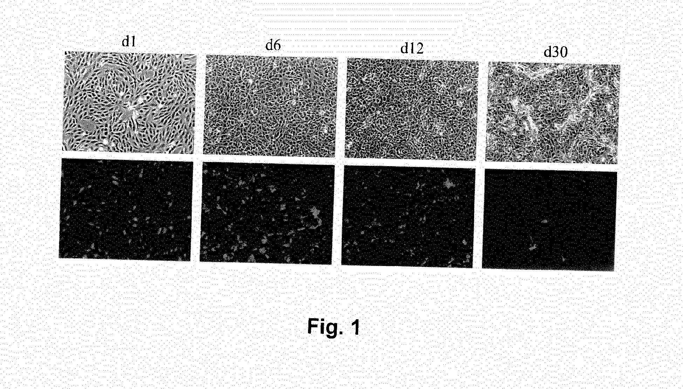

Fig. 1 show light microscopic images (upper part) and fluorescence images (lower part) of Vero cells transduced with SeV-PΔ2-77/GFP (green fluorescence protein) at day one (d1), day six (d6), day twelve (d12) and day thirty (d30) at 100x magnification.

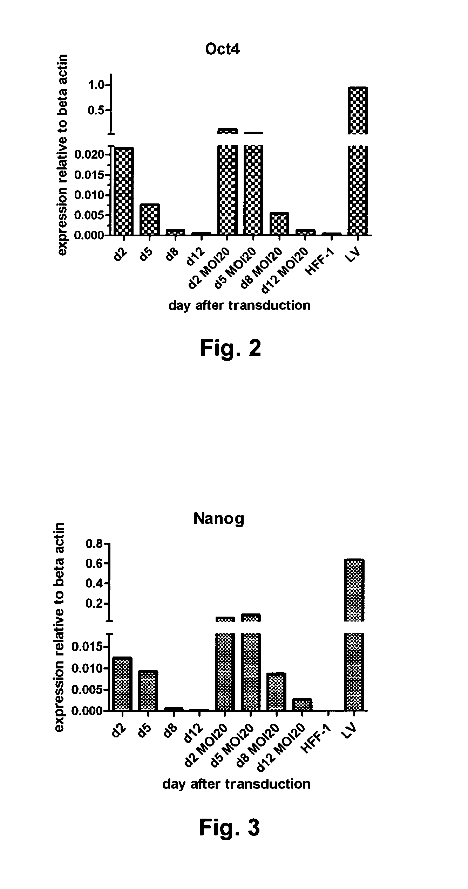

Fig. 2 is a bar chart showing the mRNA expression in human foreskin fibroblasts (HFF) transduced with SeV-PΔ2-77/Oct4 at a MOI of 3 or 20, respectively, at days two, five, eight and twelve (d2, d5, d8 and d12) in comparison to untransduced HFF cells as negative control and a recombinant lentivirus vector harbouring a GFP transgene and the Oct4 transgene as positive control.

Fig. 3 is a bar chart showing the mRNA expression in human foreskin fibroblasts (HFF) transduced with SeV-PΔ2-77/Nanog with a MOI of 3 or 20, respectively, at days two, five, eight and twelve (d2, d5, d8 and d12) in comparison to untransduced HFF cells as negative control and a recombinant lentivirus vector harbouring a GFP transgene and the Nanog transgene as positive control.

### DETAILED DESCRIPTION OF THE INVENTION

The present invention provides a method of expressing a heterologous nucleic acid sequence (sometimes referred to herein as "transgene") encoding a cellular reprogramming or programming factor or a therapeutic protein in a cell. The method is based on the use of a specific recombinant negative-strand RNA virus vector. This recombinant negative-strand RNA virus vector is designed to carry at least one heterologous nucleic acid sequence and is characterized by its deficiency of replication while still being transcription-competent to an extent that allows the efficient production of heterologous proteins of interest.

The present invention is not concern with vaccination in general and the use of the recombinant negative-strand RNA virus vector as vaccine in particular. This is, the cellular reprogramming or programming factor or a therapeutic protein encoded by the recombinant negative-strand RNA virus vector is not used, and typically not suited, for vaccination purposes.

In the method of the present invention, the recombinant replication-deficient negative-strand RNA virus vector is used for infecting a cell (sometimes referred to herein as "host cell"), thereby introducing at least one heterologous nucleic acid sequence into the cell. The introduced nucleic acid is RNA which will not integrate into the genome of the cell but will remain in the cytoplasm where it serves as a template for the viral RNA-dependent RNA polymerase (vRdRp) to generate a positive RNA strand which is decoded by the target cells' translation machinery to produce the desired heterologous proteins.

Surprisingly, it was found that the method of the present invention results in high transgene expression levels while at the same time having a superior safety profile. During infection with the replication-deficient RNA virus used in the present invention there are much less templates present compared to the wild-type RNA virus. Considering the reduced number of templates, the observed heterologous protein expression was unexpectedly high using the replication-deficient RNA virus described herein.

Furthermore, the method of the present invention eliminates the risk of malignant transformation associated with chromosomal integration since negative-strand RNA viruses are considered not to integrate into hosts' chromosomes. In addition, the risk of subsequent re-expression of chromosomally-integrated genes coding for reprogramming factors in the differentiated cells is eliminated. Also, the replication-deficient negative-strand RNA virus vector is not being transmitted to neighbouring healthy cells of the patient or from the patient to other individuals or into the environment.

In addition, the replication-deficient negative-strand RNA viral particles are evenly distributed among the daughter cells produced in each cell division cycle and, thus, are progressively diluted to undetectable levels. In other words, the replication-deficient negative-strand RNA virus vector gets completely eliminated from the infected target cells without the need for additional elaborate purification as described in the prior art.

Moreover, the replication-deficient negative-strand RNA virus vectors of the present invention bypass the risks associated with persistence of the virus vector, such as immune reactions against viral components, delayed effects of viral infection, autoimmune-like reaction to self antigens, altered expression of the endogenous host genes and unpredictable adverse events.

The RNA virus vector's ability to transcribe its RNA genome while being replication-deficient is the result of mutations in the viral P protein. The P protein is part of the viral RNA-dependent RNA polymerase (vRdRp) consisting of P and L proteins. The vRdRp carries out both viral transcription and viral replication. These two functions are therefore coupled in negative-strand RNA viruses. Mutations in the P protein were found to lead to a loss of the viral genome replication ability while the viral transcription ability, which is essential for the expression of the introduced heterologous nucleic acid sequences, is not lost. In other words, certain mutations in the P protein uncouple the replication and transcription activities of the vRdRp.

After infection of a target cell, the replication-deficient negative-strand RNA virus described herein allows for the expression of the viral proteins and heterologous proteins encoded by the at least one heterologous nucleic acid sequence included in the viral genome. In order to express the heterologous gene product, the recombinant virus used in the present invention must be capable of carrying out early primary transcription. As used herein, the term "early primary transcription" is intended to refer to the first transcriptional events in an infected host cell, where the viral RNA genome is transcribed by the vRdRp molecules that were originally included in the viral particles.

Furthermore, the recombinant virus used in the present invention must be capable of carrying out late primary transcription. The term "late primary transcription", as used herein, is intended to refer to the phase in which *de novo* proteins synthesis begins and transcription is increasingly carried out by newly synthesised vRdRp. In contrast to early primary transcription, the viral replication during late primary transcription depends on *de novo* protein synthesis. It is noted that in the prior art, but not herein, the term "secondary transcription" is sometimes used as a synonym to late primary transcription.

As soon as sufficient amounts of N protein have been produced, the vRdRp switches to a replicative mode to synthesize antigenomes. From the antigenome templates, new genomes are replicated that are encapsidated with N proteins. As the number of templates for viral transcription in the cell increases, the so-called "secondary transcription" phase begins, which exhibits a much higher transcription efficacy than the primary transcription. As used herein, the term "primary transcription" is intended to mean both early and late primary transcription, unless otherwise stated.

Preferably, the ability of the recombinant virus vector to carry out late primary transcription is such that protein synthesis is at least 1%, at least 2%, at least 3%, at least 4%, or at least 5% of the viral protein synthesis of a corresponding wild-type virus, i.e. a virus without the mutation in the gene P as described herein. The late primary transcription activity is preferably not more 20 times, more preferably not more than 10 times lower than that of a corresponding wild-type virus. The capacity for late primary transcription can be determined by quantitative determination of the expression of a heterologous gene product, e.g. a reporter protein, as described (see, e.g., Examples 7.1 and 7.3 of WO 2006/084746 A1, the disclosure of which is incorporated herein by reference).

In this connection, it should be noted that the above-mentioned heterologous expression levels are unexpectedly high in view of the fact that during infection with a (replication-competent) wild-type RNA virus there are about 500 to about 1000 times more templates present as compared to the replication-deficient RNA virus used in the present invention, which is not able to generate ribonucleoprotein complexes (RNFs) acting as templates for transcription and replication.

Within the meaning of the present invention, a "loss of replication ability" means that in a target cell, i.e. a cell that does not provide and complement any *in trans* functions that are affected by mutations in the P gene, no residual replication ability, i.e. no amplification of the viral genome, is detectable. In fact, the viral replication is considered to be entirely abolished. In contrast to decreased or conditional replication-deficiency, there are no permissive conditions that allow for viral genome replication. The loss of the capacity for replication can be determined as described in the art (see, e.g., Example 8 of WO 2006/084746 A1, the disclosure of which is incorporated herein by reference).

The recombinant negative-strand RNA virus vector used within the present invention is preferably derived from a non-segmented, negative-strand RNA virus of the order *Mononegavirales,* including wild-type strains, mutated strains, laboratory-passaged strains, vaccination strains, genetically constructed strains, and the like. As used herein, the term "virus vector" relates to virus-derived nucleic acid that can be transfected into cells and replicated within or independently of a cell genome, and includes whole viruses or viral particles, or viral cores consisting of viral genome and associated proteins. The replication-deficient, negative-strand RNA virus vector used in the present invention can be obtained by genetically modifying an initial negative-strand RNA virus.

The virus from which the recombinant negative-strand RNA virus vector is derived may be selected from negative-strand RNA viruses belonging to the families *Paramyxovirdae, Rhabdoviridae, Filoviridae, Bornaviridae, Arenaviridae* or *Bunyaviridae,* or recombinant variants thereof. Especially preferred for use in the present invention is a paramyxovirus, e.g. Sendai virus, parainfluenzavirus (PIV), e.g. human parainfluenza virus (hPIV) type 1, 2, 3, 4a or 4b, Newcastle disease virus, mumps virus, measles virus, rinderpest virus, human respiratory syncytial virus (RSV), or a rhabdovirus, e.g. vesicular stomatitis virus (VSV) or rabies virus. In an especially preferred embodiment of the invention, the virus is a Sendai virus, for example the Sendai Fushimi strain (ATCC VR105), the Sendai Harris strain, the Sendai Cantell strain or the Sendai Z strain.

A suitable recombinant negative-strand RNA virus vector may be chosen depending on the cell type to be infected and on the application. For example, Sendai virus and mumps virus show a very broad host cell specificity. Sendai virus can infect a variety of animal cells of many tissue types such as equine derived cells and B-lymphocytes of various animals. Alternatively, it may be desirable to use a negative-strand RNA viruses having high host cell specificity, for example measles virus or rinderpest virus. Viruses with artificially modified host cell specificity can also be used.

In accordance with the present invention, the mutated P protein encoded by the viral genome of the recombinant RNA virus used in the present invention contains at least one mutation that leads to a loss of the viral genome replication ability without a loss of the viral transcription ability. The at least one mutation is not restricted to a particular type of mutation and includes deletions, substitutions and/or insertions, provided that it results in a loss of the replication ability without a loss of the viral transcription ability, preferably without a loss of the early and/or late primary transcription ability.

The mutation affects the N-terminal region of the P protein of the respective negative-strand RNA virus. Preferably, the at least one mutation in the P gene is a deletion, insertion or replacement of one or more amino acids of or into the amino acid sequence 2 to 77 (numbering as, e.g., in WO 2006/084746 A1), in particular of or into the amino acid sequence 33 to 41, of the P protein of Sendai virus or, in case of a virus other than Sendai, of or into an amino acid sequence corresponding to or homologous to the amino acid sequence 2 to 77, in particular the amino acid sequence 33 to 41, of the P protein of Sendai virus.

Within the meaning of the present invention, the term "homologous" refers to a homology degree of at least 50%, 60%, 70%, 80% or 90%. In more preferred embodiments, the term "homologous" means amino acid identity values of more than 45%, 55%, 65%, 75%, 85% or 95%.

Particularly preferred is a deletion of one or more amino acids of the amino acid sequence 2 to 77 of the P protein of Sendai virus or, in case of a virus other than Sendai, of the corresponding or homologous amino acid sequence in a P protein equivalent from the virus other than Sendai virus. Most preferred is a deletion of amino acids 2 to 77 or 33 to 41 of Sendai virus or, in case of a virus other than Sendai, a deletion of the corresponding or homologous amino acid sequence in a P protein equivalent of the virus other than Sendai virus. Furthermore, the C-terminal region of the Sendai virus P protein downstream of amino acid 320, or the corresponding or homologous C-terminal region of a P protein equivalent from a virus other than Sendai, preferably includes no mutations since this C-terminal region is thought to be essential for viral transcription.

In addition to the mutations in the P gene described above, the recombinant RNA viral vector may harbor additional mutations in one or more other viral genes, which preferably decrease viral spread or viral cytotoxicity, alter the viral cell specificity or mediate attenuation of the virus. For example, the recombinant RNA viral vector may additionally have mutations or deletions in one of the genes encoding viral envelope proteins.

Also, the recombinant RNA viral vector may have one or more mutations in the C, W, and/or V open reading frames (ORFs) as a result of N-terminal deletions in the viral P protein, because the C, W, and V ORFs overlap with the N-terminal ORF of the P gene. Furthermore, the recombinant RNA viral vector used herein may additionally have a deletion of the alternative start codon ACG of the C' gene. The C' gene encodes a non-structural protein known to exhibit an anti-IFN response activity in infected cells. The deletion of the start codon of the C' gene was found to result in increased expression levels of heterologous gene products in infected target cells. Typically, the thus achievable increase in transgene expression is at least about 5%, preferably at least about 10% and more preferably at least about 20%.

In accordance with the present invention, the recombinant negative-strand RNA virus vector further comprises at least one heterologous nucleic acid sequence encoding a heterologous gene product. As used herein, the term "heterologous" is intended to refer to a protein or nucleic acid derived from a source other than the replication-deficient negative-strand RNA virus vector used within the present invention. The heterologous gene product is preferably a protein, in particular a reprogramming factor mediating or facilitating cellular reprogramming of a cell to a less-differentiated state, a programming factor that differentiates a given cell into a desired differentiated state, and a therapeutic protein. However, it may also be a ribozoyme, an antisense-molecule, a siRNA molecule, a miRNA, or a piRNA molecule. The heterologous nucleic acid sequence is typically included in the viral genome and operatively linked with appropriate expression control sequences.

As used herein, the term "reprogramming factor" or "cellular reprogramming factor" is intended to refer to a heterologous gene product, preferably a protein or a miRNA, which is capable of converting an at least partially differentiated cell to a less differentiated cell, either by itself or in combination with other genes or heterologous gene products. Also included within the term "reprogramming factor", as used herein, are auxiliary factors that facilitate cellular reprogramming (i.e. these auxiliary factors increase the efficiency of cellular reprogramming). Cellular reprogramming factors are typically proteins encoded by genes which are expressed in embryonic stem cells or in the early embryo, but are not expressed or exhibit decreased expression in the majority of differentiated somatic cells. Such embryonic stem cell specific genes are typically transcription factors and nucleoproteins.

Preferred cellular reprogramming factors for use herein are selected from the group consisting of Oct-3, Oct-4, Sox2, c-Myc, Klf4, Nanog, Lin28, ASCL1, MYT1L, TBX3b, SV40 large T, hTERT, miR-291, miR-294, miR-295, and combinations thereof.

The "programming factor" encoded by the at least one heterologous nucleic acid sequence is intended to mean a factor that is able to differentiate a given cell into a desired differentiated state. Examples include, but are not limited to, nerve growth factor (NGF), fibroblast growth factor (FGF), interleukin-6 (IL-6), bone morphogenic protein (BMP), neurogenin3 (Ngn3), pancreatic and duodenal homeobox 1 (Pdx1), Mafa, and combinations thereof.

Therapeutic proteins for use in the present invention comprise biologically active forms of proteins that are dysfunctional, typically as a result of mutations, in genetic disorders, such as adenosine deaminase (ADA) in severe combined immune deficiency, p91-PHOX in chronic granulomatus disorder, factor IX in hemophilia B, factor VIII in haemophilia A, cystic fibrosis transmembrane conductance regulator (CFTR) in cystic fibrosis, β-globin (HBB) in sickle cell anemia, dystrophin in Duchenne muscular dystrophy, hypoxanthine-guanine phosphoribosyl-transferase (HGPRT) in Lesch-Nyhan syndrome, phenylalanine hydroxylase (PAH) in phenylketonuria, glucosylceramidase (GBA and GBA2) in Gaucher's disease, fibrillin-1 (FBN1) in Marfan syndrome, and huntingtin (HTT) in Huntington's disease, and combinations thereof.

The method of the present invention may also be used for the treatment of multifactorial genetic disorders by delivery of one or more therapeutic genes. For example, therapeutic genes for the treatment of hypercholesterolemia include apolipoprotein B (apoB), low density lipoprotein receptor (LDLR), low density lipoprotein receptor adaptor protein 1 (LDLRAP1), proprotein convertase subtilisin/kexin type 9 (PCSK9), and combinations thereof. Furthermore, therapeutic genes for the treatment of Parkinson's disease include synuclein alpha (SNCA), parkin (PRKN), leucine-rich repeat kinase 2 (LRRK2), PTEN induced putative kinase 1 (PINK1), parkinson protein 7 (DJ-1), and ATPase type 13A2 (ATP13A2). In addition, therapeutic genes for the treatment of cancer include cancer suicide genes, anti-angiogenic factors, and cancer self antigens, including tyrosinase-related protein 2 (TRP-2) and carcinoembryonic antigen (CEA).

Suitable therapeutic proteins, in particular for use in cancer immunotherapy, may further include immune stimulating factors, such as growth factors including granulocyte-macrophage colony-stimulating-factor (GM-CSF) and epithelial growth factor (EGF), and cytokines including interleukins IL-2, IL-4, IL-5, IL-12, and IL-17, as well as immunosuppressive proteins such as interferons.

The heterologous nucleic acid sequences incorporated in the replication-deficient RNA virus vector may be derived from humans or other mammals depending on the application and the target cell into which said nucleic acid sequences are to be delivered. The nucleic acid sequences and the encoded amino acid sequences do not necessarily have to be wild-type sequences as long as the heterologous gene products show functional activity comparable to the wild-type gene product. The nucleic acid sequences may harbor nucleotide exchanges, insertions or deletions. The heterologous gene products may be expressed as fusion proteins, e.g. additionally comprising a tag, preferably a VP16 tag.

If high expression of the heterologous nucleic acid sequence is desired, the sequence is preferably inserted into the 3' region of the viral negative-strand RNA genome, preferably directly before the viral N gene. The reason is that negative-strand RNA viruses like paramyxoviruses most efficiently transcribe transcription units at the 3' end of their negative-strand genome. Transcript levels of genes further downstream gradually decrease, a phenomenon that is referred to as polarity effect. This allows regulating the expression level of a heterologous transgene by inserting it at different sites in the viral genome. Hence, a nucleic acid sequence encoding a heterologous gene product with cytotoxic or oncogenic potential (e.g. c-Myc) may be inserted into the 5' region of the viral negative-strand genome. Conveniently, heterologous nucleic acid sequences may be inserted as transcriptional cassettes. Several heterologous nucleic acid sequences may be inserted as independent transcriptional cassettes into the viral genome. A transcriptional cassette usually comprises the nucleic acid sequence encoding the heterologous gene product operatively linked to a transcription start sequence, a transcriptional terminator, and preferably translation signals.

It is also possible to operatively link a heterologous nucleic acid sequence with an mRNA stabilizing element. For instance, a Woodchuck hepatitis virus post-trancriptional regulatory element (WPRE) may be inserted into the 3'UTR region of the transgene in order to stabilize its mRNA and prolong its expression.

In the context of the present invention, the replication-deficient negative-strand RNA virus vector may harbor more than one heterologous nucleic acid sequence encoding a heterologous gene product. Alternatively, more than one replication-deficient negative-strand RNA virus vectors, each of which harbors at least one heterologous nucleic acid sequence encoding a heterologous gene product, can be used concurrently. For instance, the replication-deficient negative-strand RNA virus vector can comprise several nucleic acid sequences encoding different reprogramming factors. Alternatively, multiple replication-deficient negative-strand RNA virus vectors harboring different nucleic acid sequences encoding different reprogramming factors can be used simultaneously.

The replication-deficient negative-strand RNA virus vector can be provided in the form of a solution, suspension, lyophilisate, or in any alternative form. It can also be provided in combination with pH-adjusting agents, buffers, agents for the adjustment of toxicity such as sodium chloride or dextrose, wetting agents, adjuvants, and the like.

The appropriate virus vector dose depends on the intended application, the physical condition, weight, age and sex of the patient, the form of administration, and the composition. Typically, the number of vector particles varies from at least 1 x 10⁴ to 1 x 10⁸, preferably from 5 x 10⁵ to 1 x 10⁷, per dose depending on the application. In other words, infection with the recombinant viral vector of the invention will preferably be carried out with a multiplicity of infection (MOI) of from 0.01 to 50, more preferably from 0.5 to 30, depending on the application.

The replication-deficient negative-strand RNA virus vectors of the present invention can be prepared as described in WO 2006/084746 A1, the disclosure of which is incorporated herein by reference, by means of virus-producing cells and virus-amplifying cells.

Virus-producing cells are used for the initial production of viral particles. The virus-producing cell is preferably a eukaryotic cell, more preferably a mammalian cell. The virus-producing cell preferably comprises a DNA molecule encoding the replication-deficient RNA virus vector used in the present invention, as well as helper sequences whose gene products allow for the assembly of the recombinant viral particles of the present invention *in trans.* The helper sequences comprise the viral N protein, the viral P protein, and/or the viral L protein, preferably the viral N protein and viral P protein. The helper cell can comprise one or several additional plasmid vectors, which provide the viral N protein, the viral P protein, and/or the viral L protein *in trans.* Preferably, the helper sequences comprise the viral P gene and additionally the viral N gene since it was surprisingly found that the production of viral particles can be significantly increased by coexpressing the viral N and P genes. Alternatively, a helper cell stably expressing helper sequences which are integrated in its genome may be used.

The helper sequences are preferably operatively linked with a transcriptional signal that allows transcription by a DNA dependent RNA polymerase in the virus-producing cell. Preferably, the transcriptional signal is a heterologous transcriptional signal for the given virus-producing cell, e.g. a bacteriophage promoter such as a T7 or a SP6 promoter. In this case the virus-producing cell must comprise the according heterologous DNA dependent RNA polymerase, e.g. the T7 or the SP6 RNA polymerase, which mediates transcription of the helper sequences.

The DNA molecule encoding the replication-deficient RNA virus used in the present invention is preferably a vector (e.g., a plasmid vector) that is not only suitable for propagation in a vector amplifying cell (i.e. in a prokaryotic vector amplification cell like *E. coli*), but also in a eukaryotic helper cell, in particular in a mammalian virus-producing cell. The vector comprises genetic elements necessary for propagation in said cells, such as an origin of replication, and/or selection marker sequences.

The DNA molecule encoding the viral genome is commonly operatively linked with a transcriptional signal as described above in connection with the helper sequences. Generally, the DNA molecule further comprises a transcriptional terminator and a ribozyme sequence at the 3' end of the DNA sequence encoding the viral genome. The ribozyme sequence allows for cleavage of the transcript at the 3' end of the viral sequence.

The virus-amplifying cell is used for amplifying the virus particles initially assembled in the virus-producing cell. The recombinant replication-deficient RNA virus obtained from the virus-producing cell is used for infecting the virus-amplifying cell. The virus-amplifying cell contains helper sequences as described above, which provide the viral N protein, the viral P protein, and/or the viral L protein. Preferably, a virus-amplifying cell stably expressing helper sequences which are integrated in its genome is used. Preferably, the virus-amplifying cells are genetically modified to express only the viral N and P proteins, but not the viral L protein, since it was surprisingly found that this coexpression leads to the highest virus production rates.

Preferably, the virus-amplifying cell is a mammalian cell. In particular, the virus-amplifying cell may be the H29 cell deposited at the German Collection of Microorganisms and Cell Cultures under accession number DSMACC2702. Other suitable virus-amplifying cells are cells derived from Vero cells (an African green monkey kidney cell line) or cells derived from LLCMK2 cells (a Rhesus monkey kidney cell line), which are stably or transiently transfected with the mentioned helper sequences (i.e. the viral N, P, and/or L gene).

In accordance with the method of the present invention, a variety of different cells can be infected with the replication-deficient negative-strand RNA virus vector in order to introduce the at least one heterologous nucleic acid sequence. Examples of cells that may be infected with the replication-deficient negative-strand RNA virus vector include, but are no limited to, human foreskin fibroblasts, human hematopoietic stem cells (CD34+ cells), dendritic cells, T cells, B cells, macrophages, cells of mucosal tissue, hepatocytes, lung fibroblasts, and epithelial cells.

The cell to be infected may be obtained or derived from any host organism. The cell may be directly taken from a respective host organism in form of a sample, such as a biopsy or a blood sample. It may also be a cell that has been obtained from a host organism and subsequently been cultured, grown, transformed or exposed to a selected treatment. In some embodiments, the cell may be included in a host organism. For instance, it may be present in the blood or in an organ of the host organism.

The host organism from which the cell is derived or obtained is preferably a mammal and especially preferred a human. Examplary mammals are selected from, but are not limited to, the group consisting of rat, mouse, rabbit, guinea pig, squirrel, hamster, vole, platypus, dog, goat, horse, pig, elephant, chicken, macaque, and chimpanzee.

In a preferred embodiment, the method of the present invention is an *in vitro* method of reprogramming an at least partially differentiated cell to a less differentiated cell or programming a cell to be programmed to a desired differentiated state, the method comprising:
(a) providing an at least partially differentiated cell or a cell to be programmed from a donor,
(b) introducing at least one heterologous nucleic acid sequence into the cell provided in step (a) by infecting the cell with a recombinant negative-strand RNA virus vector comprising said at least one heterologous nucleic acid sequence,and
(c) culturing the infected cell under conditions effective to express said at least one heterologous nucleic acid sequence,
wherein the recombinant negative-strand RNA virus vector includes a viral genome coding for a mutated P protein, which leads to a loss of the viral genome replication ability without a loss of the viral transcription ability, and wherein said heterologous nucleic acid sequence encodes a cellular reprogramming or programming factor.

The term "differentiated", as used herein, is intended to refer to a cell exhibiting a restricted potency as compared to pluripotent stem cells. The term "potency" means the differentiation potential of a cell, this is the potential of a cell to differentiate into different cell types. Thus, in the context of the present invention differentiated cells represent all cells that are more differentiated than pluripotent stem cells and include cells that still possess the ability to differentiate into multiple, but not all, cell lineages. Differentiated cells include, in particular, somatic cells. Similarly, the term "differentiation", as used herein, is intended to refer to the adaptation of cells to a particular form or function. Differentiation leads to a more committed cell, i.e. a cell that is considered to be permanently committed to a specific function, such as a terminally differentiated cell.

Furthermore, the term "reprogramming" or "dedifferentiation", as used herein, refers to the conversion of the differentiated state of a particular cell to a less differentiated state. The terms "reprogramming" and "dedifferentiation" are used interchangeably herein and refer to a loss of specialization in form or function. Dedifferentiation leads to a less committed cell. Thus, it refers to increasing the degree of potency of a particular cell and includes converting a terminally differentiated cell to an oligopotent, preferably to a multipotent, or more preferably to a pluripotent cell. It also includes converting the differentiation state of a cell exhibiting restricted potency as compared to pluripotent cells, such as an oligopotent or a multipotent cell, to a less differentiated state. In the context of the present invention reprogramming or dedifferentiation means reverting a cell other than a pluripotent stem cell to its initial state or any intermediate state in its path of differentiation.

Within the context of the present invention, an "at least partially differentiated cell", as used herein, refers to a cell that builds up the body of a multicellular organism, excluding a gamete, a germ cell, a gametocyte or a pluripotent stem cell. Examples of partially differentiated cells include, for example, somatic stem cells, lineage-restricted stem cells, precursor cells, and progenitor cells. Examples of (more) differentiated cells include somatic cells, such as terminally differentiated somatic cells. Preferably, the at least partially differentiated cell is a mammalian cell selected from a somatic stem cell, a lineage-restricted stem cell, a precursor cell, a progenitor cell, and a terminally differentiated somatic cell.

The term "stem cell", as used herein, refers to a cell of multicellular organisms that can proliferate infinitely and has the capacity to self-renew and differentiate into diverse specialized cell types. Stem cells include embryonic stem cells (i.e. pluripotent stem cells derived from the inner cell mass of blastocysts) and adult stem cells also known as "somatic stem cell" (i.e. multipotent stem cells found in various tissues that act as repair system for the body, replenishing adult tissues). Examples include, but are not limited to, mesenchymal stem cells, hematopoietic stem cells, and neural stem cells. Also included are so-called cancer stem cell. Many types of cancer have been found to include such cancer stem cells, which are characterized by their self-renewing capacity and differentiation ability. Cancer stem cells resemble the progenitor from which they arose, but express a self-renewal-associated programme normally expressed in stem cells.

A "precursor cell" within the meaning of the present invention is a stem cell that has developed to a stage where it is committed to differentiating into one or more final forms. Precursor cells are for instance committed to form a particular kind of new blood cells, lineage-restricted stem cells, or somatic stem cells.

A "progenitor cell" within the meaning of the present invention is an unipotent or multipotent cell, which has the capacity to differentiate into a specific type of cell, for instance a mature somatic cell, and has a limited ability of self-renewal. Examples of suitable progenitor cells include, but are not limited to, neuronal progenitor cells, endothelial progenitor cells, erythroid progenitor cells, cardiac progenitor cells, oligodendrocyte progenitor cells, retinal progenitor cells, or hematopoietic progenitor cells.

A "somatic cell" within the meaning of the present invention is any at least partially differentiated cell and does not include germ cells or gametes. A somatic cell for use in the present invention may be a cell of any tissue, including skin, kidney, spleen, adrenal glands, liver, lung, ovary, pancreas, uterus, stomach, colon, small intestine, spleen, bladder, prostate, testicles, thymus, muscle, connective tissue, bone, cartilage, vascular tissue, heart, eye or neural tissue. Examples of suitable somatic cells include, but are not limited to, fibroblasts, myeloid cells, B lymphocytes, T lymphocytes, bone cells, bone marrow cells, pericytes, dendritic cells, keratinocytes, adipose cells, mesenchymal cells, epithelial cells, epidermal cells, endothelial cells, chondrocytes, cumulus cells, neural cells, glial cells, astrocytes, cardiac cells, esophageal cells, muscle cells (e.g. smooth muscle cells or skeletal muscle cells), pancreatic beta cells, melanocytes; hematopoietic cells, myocytes, macrophages, monocytes, and mononuclear cells.

A "terminally differentiated somatic cell" within the meaning of the present invention is a cell at the end stage of a differentiation pathway. A terminally differentiated cell is a mature cell that has undergone progressive developmental changes to a more specialized form or function. Differentiated cells have distinct characteristics, perform specific functions, and are less likely to divide than their less differentiated counterparts.

The at least partially differentiated cell, which is reprogrammed in accordance with the method of the present invention, is not particularly limited to a specific organism and include, but are not limited to, the organisms mentioned herein above. Also, the type of cell is not particularly limited and includes, but is not limited to, the cells mentioned above. Preferably, the cell to be reprogrammed is a terminally differentiated somatic cell.

Within the meaning of the present invention, the "less differentiated cell", which is generated by the *in vitro* reprogramming method of the present invention, is a cell having an increased degree of potency compared to the original cell that is at least partially differentiated. Preferably, the *in vitro* method of reprogramming of an at least partially differentiated cell to a less differentiated cell is characterized in that the at least partially differentiated cell is reprogrammed to an oligopotent, multipotent or pluripotent cell.

A "pluripotent cell" means a cell having the potential to differentiate into the three germ layers ectoderm, mesoderm and endoderm. "Pluripotent" refers to a degree of potency lower than omnipotency (the potential of a cell to give rise to all cells of an organism).

A "multipotent cell" means a cell having the potential to differentiate into cells of multiple but limited number of lineages. It refers to a degree of potency lower than pluripotency. Multipotent stem cells are stem cells differentiating normally into only cell types specific to their tissue and organ of origin. Multipotent stem cells are involved not only in the growth and development of various tissues and organs during the fetal, neonatal and adult periods but also in the maintenance of adult tissue homeostasis and the function of inducing regeneration upon tissue damage. Tissue-specific multipotent cells are collectively called "adult stem cells".

An "oligopotent cell" means a cell having the potential to differentiate into a few cell types. "Oligopotent" refers to a degree of potency lower than multipotency.

Preferably, the less differentiated cells generated by the method of repogramming of the present invention are "induced pluripotent stem (iPS) cells". In the context of the present invention, the term "induced pluripotent stem cell" or the term "iPS cell" is intended to refer to a type of pluripotent stem cell artificially derived from an at least partially differentiated cell, i.e. a non-pluripotent cell, typically a somatic cell, by heterologous expression of specific stem cell associated reprogramming factors iPS cells are similar to natural pluripotent stem cells, such as embryonic stem cells, with respect to many aspects, such as the stem cell associated gene expression patterns, the chromatin methylation patterns, the doubling time, the embryoid body formation, the teratoma formation, the viable chimera formation, the potency and the differentiability.

The term "pluripotent stem cell", as used herein, refers to a stem cell having the potential to differentiate into the three germ layers ectoderm, mesoderm and endoderm. "Pluripotent stem cells" include natural pluripotent stem cells like embryonic stem (ES) cells and induced pluripotent stem (iPS) cells. Embryonic stem cells are derived from the inner cell mass located inside of blastocysts and have the ability to differentiate into any cell type, except the cells of the placenta or other supporting cells of the uterus, and cannot therefore form new living organisms.

Pluripotency may be evaluated by the ability of cells to form a chimera, a blend of cells from two or more organisms, after combining the respective stem cells or stem-like cells with the blastocyst of an embryo that subsequently forms a completely integrated organism from the cell mixture. A further way of evaluating pluripotency is the injection of the respective stem cells beneath the skin of a mouse where they can form a teratoma. A further evaluation method is tetraploid complementation, an *in vivo* test that measures the pluripotency of corresponding cells by their injection into 4N embryos that are incapable of further differentiation. The resultant normal 2N embryo continues to develop from the imported pluripotent cells.

The differentiation status of a cell can also be assessed microscopically based on the phenotype displayed by the cell. Raman microspectroscopy or FT-IR spectroscopy are suitable techniques for assessing the differentiation status in this regard. Alternatively, the differentiation status of a cell can be assessed by measuring the amount of a marker of the differentiation status of a cell, typically a cellular protein. The differentiation marker may be detected on mRNA or protein level using various techniques such as microarray hybridization or antibody staining. Generally, it is advantageous to select a combination of several markers for assessing the differentiation status of a cell.

Examples of marker proteins of the differentiation status of a cell include, but are not limited to, Nanog, Oct-3/4, Sox2, Sall4, Tcll, Tbx3, Eras, Klf2, Klf4, Klf5, Baf250a, BCO31441, Eno3, Etv5, Gm1739, Gtf2h3, Hes6, Jub, Mtf2, Myodl, Nmycl, Notch4, Nr5a2, Nrg2, Otx2, Rab2b, Rbpsuh, Rest, Stat3, Utfl, Tcfap2c and Zfp553, or the methylation status of the promoter of one of Nanog, Oct4, Sox2, Sall4, Tcl 1, Tbx3, Eras, Klf2, Klf4, Klf5, Baf250a, BC031441, Eno3, Etv5, Gm1739, Gtf2h3, Hes6, Jub, Mtf2, Myodl, Nmycl, Notch4, Nr5a2, Nrg2, Otx2, Rab2b, Rbpsuh, Rest, Stat3, Utf1, Tcfap2c, and Zfp553.

iPS cells prepared according to the present invention typically express alkaline phosphatase, a marker of ES-like cells. Further, iPS cells of the present invention usually express endogenous Oct-3/4 or Nanog, in particular both Oct-3/4 and Nanog. Moreover, they preferably express TERT, and show telomerase activity.

Within the present invention, a combination of different reprogramming factors may also be used to induce cellular reprogramming of a cell to be reprogrammed. Several genes encoding reprogramming factors may be inserted into the same replication-deficient RNA virus vector. Alternatively, different nucleic acid sequences encoding different reprogramming factors may each be inserted into separate replication-deficient virus vectors. The replication-deficient RNA virus vector used in the present invention may also be used in combination with one or more vectors of a different type harboring one or more heterologous nucleic acid sequences encoding heterologous proteins of interest.

The reprogramming factors expressed from the replication-deficient RNA virus vector employed herein may be used in combination with compounds that facilitate cellular reprogramming, including bFGF, SCF, MAP kinase inhibitors, DNA methylase inhibitors (e.g., 5-azacytidine), and histone deacetylase inhibitors (e.g., suberoylanilide hydroxamic acid (SAHA), trichostatin A (TSA), and valproic acid (VPA)). The addition of these compounds can also limit the number of reprogramming factors necessary for induction of cellular reprogramming. Furthermore, the delivery of reprogramming factors into somatic cells using the replication-deficient RNA virus may be combined with chemical treatment of the somatic cell to induce endogenous expression of additional endogenous reprogramming factors.

In the *in vitro* programming method of the present invention, the cells to be programmed to a desired differentiated state are terminally differentiated somatic cells including, but not limited to, the cells mentioned hereinabove, i.e. human foreskin fibroblasts, human hematopoietic stem cells (CD34+ cells), dendritic cells, T cells, B cells, macrophages, cells of mucosal tissue, hepatocytes, lung fibroblasts, and epithelial cells.

Within the context of the present invention, the cells obtained by the *in vitro* method of reprogramming, typically stem-like cells, may be used to obtain particular differentiated cells of interest. Such cells, like the cells obtained by the *in vitro* programming method of the present invention, may, for example, be used in regenerative medicine with the advantage that cells from the same individual can be used to provide cells of a selected cell type. As an illustrative example, human hematopoietic stem cells may be used in medical treatments requiring bone marrow transplantation. Such cells may also be used in the formation of one or more cell lines.

Cells obtained according to the invention are expected to be suitable for use in treating many physiological conditions and diseases, for example neurodegenerative diseases like multiple sclerosis, late stage cancers like ovarian cancer and leukemia, and diseases that compromise the immune system, such as HIV infection ("AIDS"). Further examples of physiological conditions that may be treated include, but are not limited to, spinal cord injuries, multiple sclerosis, muscular dystrophy, diabetes, liver diseases, i.e., hypercholesterolemia, heart diseases, cartilage replacement, burns, foot ulcers, gastrointestinal diseases, vascular diseases, kidney disease, urinary tract disease, and aging related diseases and conditions.

In another preferred embodiment, the method of expressing a heterologous nucleic acid sequence in a cell is an *in vivo* method of treating a genetic disorder, the method comprising administering to a patient a recombinant negative-strand RNA virus vector comprising at least one heterologous nucleic acid sequence to introduce said at least one heterologous nucleic acid sequence into a cell of the patient, wherein the recombinant negative-strand RNA virus includes a viral genome coding for a mutated P protein, which leads to a loss of the viral genome replication ability without a loss of the viral transcription ability, and wherein said heterologous nucleic acid sequence encodes a therapeutic protein capable of treating the genetic disorder.

In a further preferred embodiment, the method of expressing a heterologous nucleic acid sequence in a cell is an *in vitro* method of treating a genetic disorder, the method comprising:
(a) providing a cell from a donor,
(b) introducing at least one heterologous nucleic acid sequence into the cell provided in step (a) by infecting the cell with the recombinant negative-strand RNA virus vector comprising said at least one heterologous nucleic acid sequence,and
(c) culturing the infected cell under conditions effective to express said at least one heterologous nucleic acid sequence and/or to allow the cell to expand,
wherein the recombinant negative-strand RNA virus vector includes a viral genome coding for a mutated P protein, which leads to a loss of the viral genome replication ability without a loss of the viral transcription ability, and wherein said heterologous nucleic acid sequence encodes a therapeutic protein capable of treating the genetic disorder.

The genetic disorder to be treated by the *in vivo* and *in vitro* methods of the present invention is preferably selected from the group consisting of severe combined immune deficiency, chronic granulomatous disorder, hemophilia A/B, cystic fibrosis, sickle cell anemia, Duchenne muscular dystrophy, Lesch-Nyhan syndrome, phenylketonuria, Gaucher's disease, Marfan syndrome, Huntington's disease, hypercholesterolemia, Parkinson's disease, DiGeorge syndrome, Alzheimer's disease, and cancer.

Gene therapy approaches may be carried out *in vitro* or *in vivo,* depending on the genetic disorder to be treated and the cell type to be infected. For *in vivo* gene therapy approaches, the replication-deficient negative-strand RNA virus vector can be administered in the usual manner, e.g. orally, topically, nasally, pulmonally, etc. in the form of aerosoles, solutions, powders, and the like. *In vivo* gene therapy approaches may target genetic disorders affecting blood cells. In this case the replication-deficient negative-strand RNA virus vector may be administered parenterally. If lung cells are to be targeted, the pharmaceutical composition comprising the replication-deficient negative-strand RNA virus vector may be administered pulmonally.

In another aspect, the present invention relates to a cell or a population of cells prepared by the *in vitro* methods of the present invention, in particular the *in vitro* method of reprogramming or programming a cell and the *in vitro* method of treating a genetic disorder.

The cell or population of cells may be used as a medicament for the treatment of a variety of diseases. As mentioned above, the reprogrammed cells obtained by the *in vitro* method of reprogramming according to the present invention, may be differentiated to a desired cell type and then used in the treatment of a given disease. In particular, iPS cells generated by the *in vitro* method of reprogramming can be used in regeneration therapies as well as in basic research. Patient-specific iPS cells generated in accordance with the present invention are, for example, suited for use in stem cell therapy. The somatic cells collected from a patient are first reprogrammed to a less differentiated state using the *in vitro* reprogramming method of the present invention, differentiated into the desired somatic cell type according to procedures known to a person skilled in the art (e.g., by treatment with retinoic acid, growth factors, cytokines, and hormones), and then administered to the patient.

In another aspect the present invention relates to a pharmaceutical composition comprising the cell or the population of cells of the present invention, or a redifferentiated cell or a population of redifferentiated cells derived from the cell or population of cells prepared by the in vitro reprogramming method of the present invention. Means for differentiating a dedifferentiated cell are known in the art and also indicated hereinabove.

The pharmaceutical composition of the present invention may be in the form of a solution, a suspension or any other form suitable for the intended use. Typically, the composition further comprises a pharmaceutically acceptable carrier, diluent, and/or excipient. Agents for adjusting the pH value, buffers, agents for adjusting tonicity, and the like may also be included. The composition may be administered by the usual routes. A therapeutically effective dose of the virus is administered to the patient, which dose depends on the particular application (e.g. regeneration therapy or gene therapy), on the type of disease, the patient's weight, age, sex and state of health, the manner of administration and the formulation etc. Administration can be single or multiple, as required.

The pharmaceutical composition of the present invention is suitable for applications in human and/or veterinary medicine. It may be used for regeneration therapies as well as in gene therapy. In particular, the pharmaceutical composition of the present invention can be used in antitumor therapy.

The present invention will now be further illustrated by the following examples.

### EXAMPLES

The following shows that the use of the method of the present invention enables the efficient and long-term transgene expression in a safe and reliable way using a recombinant negative-strand RNA virus vector. These unexpected and advantageous characteristics associated with the present invention open up new fields of application for these type of virus vectors that hitherto have not been considered to be useful in the treatment of medical conditions.

### Example 1

### Long-term transgene expression

Vero cells were infected with a recombinant Sendai virus (SeV) having a viral genome that contains a mutated P gene coding for a truncated P protein lacking the N-terminal amino acids 2 to 77 as well as green fluorescence protein (GFP) as an overall marker of gene expression (SeV-PΔ2-77/GFP) at a multiplicity of infection (MOI) of 1. Fluorescence and light micrographs (exposition time of two seconds) were taken at days 1, 6, 12 and 30 at 100x magnification.

As shown in Fig. 1, the level of detectable GFP only moderately declined over a time period of no less than 30 days and was still considerably high after 30 days. Thus, the recombinant negative-strand RNA virus vector used in the present invention unexpectedly allows for the long-term expression of transgenes. This offers various new fields of application such as *ex vivo* and *in vivo* gene therapeutic approaches, reprogramming cells to a cell of a less differentiated state suitable for use in regeneration therapies, and programming cells to cells of a specifically differentiated cellular state for use in the treatment of various diseases.

### Example 2

### Real-time monitoring of heterologous mRNA expression

A recombinant replication-deficient Sendai virus having the transcription factor "Oct4" included in its viral genome as a transgene (SeV-PΔ2-77/Oct4) or a recombinant replication-deficient Sendai virus having the transcription factor "Nanog" included in its viral genome as a transgene (SeV-PΔ2-77/Nanog) was used to infect human foreskin fibroblasts (HFF) with a MOI of 3 or 20. At days 2, 5, 8, and 12, total cellular RNA was extracted using Triazol (Invitrogen) and a real-time RT-PCR was performed. The cDNA synthesis step was performed using an oligo-(dT)12-18 primer allowing amplification of only mRNA (enzyme "superscript II"; Invitrogen).

The real-time RT-PCR was carried out in accordance with procedures known to a person skilled in the art. In brief, a qPCR SYBR Green-Mix (ABgene, Surrey, UK) was used. The starting amount of cDNA was calculated by comparing the threshold cycle (C_{T}) values of each sample with C_{T} values of the respective standard curve. For this calculation the software "Mastercycler ep realplex" (Eppendorf) was used. For normalization, expression levels of the target genes were compared to beta actin transcript levels. Untransduced HFF cells served as negative controls.

Real-time PCR conditions were as follows: initial denaturation at 95 °C for 10 minutes, 40 cycles of denaturation (95 °C, 15 seconds), annealing (see table below for the respective annealing temperatures, 60 seconds), primer extension phase (72°C, 60 seconds). The existence of the correct product length was controlled by means of gel electrophoresis. The following primer pairs were used:

| | Forward | Reverse | Annealing Temperature |
|---|---|---|---|
| Beta actin | CAAGAGATGGCCACTGCC (SEQ ID NO:1) | CTTGATCTTCATGGTGCTAGGA (SEQ ID NO:2) | 55 °C |
| Nanog | GGACAGGTTTCAGAAGCAGAAG (SEQ ID NO:3) | ACCATTGCTAGTCTTCAACCAC (SEQ ID NO:4) | 59 °C |
| Oct4 | ATCCTCCCTTTATCCAGCCC (SEQ ID NO:5) | AGAAGGCGAAGTCTGAAGCC (SEQ ID NO:6) | 60 °C |

Non-transduced HFF cells served as negative control, defining the lowest level of mRNA expression (as defined here at "0.000"). As a positive control, a lentivirus vector harbouring a GFP transgene and expressing the Oct4 transgene and Nanog transgene, respectively, was used. The expression level is expressed relative to that of beta actin which was determined in parallel. The results are shown in Figs. 2 and 3.

As can be seen from Fig. 2, the transduction with a MOI of 3 (first four time points in Fig. 2) as well as the transduction with a MOI of 20 (indicated in Fig. 2 by "MOI20") resulted in an mRNA expression level of Oct4 which was still very high after 5 days (roughly about 30% for a MOI of 3 and more than 99% for a MOI of 20). Similarly, as shown in Fig. 3, the expression level of Nanog was still very high after 5 days (roughly about 75% for a MOI of 3 and essentially 100% for a MOI of 20). Even after 8 days, there was observed a significant Oct4 and Nanog mRNA expression.

The observed long-term expression of Oct-4 and Nanog from the replication-deficient Sendai vector harboring the deletion in the P protein of amino acids 2 to 77 was a surprising finding. Heretofore, the half-life of Sendai virus nucleocapsids was reported to be roughly about 24 hours (see Mottet et al., Virology 176: 1-7 (1990)). Based on this value, it was the general belief in the art that negative-strand RNA viruses, such as Sendai virus, are not suitable for many applications.

However, contrary to the general belief in the art, it was unexpectedly found that the recombinant negative-strand RNA virus allows for the long-term expression of transgenes. Moreover, the recombinant negative-strand RNA virus was also found to achieve surprisingly high expression levels in view of the fact that during infection there are about 500 to about 1000 times less templates present as compared to the wild-type virus.

### Example 3

### Elimination of C' protein from Sendai P gene

In an attempt to further enhance the long-term gene expression using the viral vector SeV-PΔ2-77, a genetic modification within the P gene was investigated. More specifically, the Sendai P gene encodes not only the P protein but several other non-structural proteins known as "C proteins". These proteins are translated from an alternative reading frame (position -1 relative to the P protein) or result from the use of an alternative start codon for translation (AUG (ATG) instead of ACG). This alternative start codon use is believed to result in a reduced gene expression rate (reduction of about 10-20% compared to the wild-type) of the P protein. In the literature, the C proteins are described to act as regulatory factors in the host virus interaction, mainly as interferon response antagonists.

In the experiment described here, the start codon of the C' protein in the viral vector genome of SeV-PΔ2-77 was eliminated by site-directed mutagenesis. More specifically, the start codon "ACG" was changed to the non-sense codon "UCU" or similar non-sense codons. Even though the skilled person would have expected that the viral vector will now be eliminated from the cell faster due to the earlier onset of an interferon response, it was surprisingly found that the transgene expression was increased (20-40%) while the survival time of the vector inside the cell was not shortened.

### Example 4

### Evaluation of an optimal production system for a replication-deficient Sendai virus vector

Comparative studies were carried out to evaluate the optimal production system for a replication-deficient Sendai vector harboring the deletion of amino acids 2-77 within the P gene. More specifically, three different settings were compared:
(1) Production cell line with only the P gene as additional transgene. This should be sufficient to produce high level amounts of the vector since all other viral components (proteins) are still encoded in their wild-type origin in the genome of the vector.
(2) Production cell line with stable integration of the Sendai virus P and N genes. This combination was expected to result in an imbalance of the viral proteins during production of the vector. Normally N and P proteins are produced at roughly equal level in the host cell (see Homann et al., Virology 177: 131-140 (1990) and Tokusumi et al., Virus Research 86: 33-38 (2002)). This ratio is regarded as critical for optimal virus growth. In this setting however, it was expected to produce the viral N protein in large excess which should result in lower production efficacy as N is known to be cytotoxic due to its inherent activity to bind RNA molecules.
(3) Production cell line with stable integration of the Sendai virus P and N and L genes. This combination reflects all viral protein components of the nucleocapsid. Therefore, it could have a supportive impact on virus production. However, as already mentioned for setting (2), it could also result in an imbalance of the viral components N and L, which are already encoded in the viral genome and thus expressed during production from the viral genome.

Surprisingly, setting (2) was identified as the best combination for production of the viral vector. Even though setting (3) was equally well with respect to the amount of vector that could be produced, setting (2) offers the advantage that only two genes have to be introduced into the cell rather than three as in setting (3).

## Claims

1. A method of expressing at least one heterologous nucleic acid sequence in a cell, the method comprising
introducing at least one heterologous nucleic acid sequence into a cell by infecting said cell with a recombinant negative-strand RNA virus vector comprising said at least one heterologous nucleic acid sequence,
wherein the recombinant negative-strand RNA virus vector includes a viral genome coding for a mutated P protein, which leads to a loss of the viral genome replication ability without a loss of the viral transcription ability, and
wherein said at least one heterologous nucleic acid sequence encodes a cellular reprogramming or programming factor or a therapeutic protein.

2. The method of claim 1, wherein said recombinant negative-strand RNA virus vector is a paramyxovirus including Sendai virus, parainfluenzavirus, Newcastle disease virus, mumps virus, measles virus, rinderpest virus and human respiratory syncytial virus, or a rhabdovirus vector including vesicular stomatitis virus or rabies virus.

3. The method of claim 1 or 2, wherein the mutation of the P protein is a deletion, insertion or replacement of one or more amino acids of or into the amino acid sequence 2-77 of the P protein of Sendai virus or, in case of a virus other than Sendai, of or into an amino acid sequence homologous to the amino acid sequence 2-77 of the P protein of Sendai virus.

4. The method of claim 3, wherein the mutation is a deletion of
(a) amino acids 2 to 77 of the P protein of Sendai virus or, in case of a virus other than Sendai, a deletion of the amino acids homologous to amino acids 2-77 of the P protein of Sendai virus or
(b) amino acids 33-41 of the P protein of Sendai virus or, in case of a virus other than Sendai, a deletion of the amino acids homologous to amino acids 33-41 of the P protein of Sendai virus.

5. The method of any one of claims 1 to 4, wherein the cellular reprogramming factor is selected from the group consisting of Nanog, Oct-3/4, Sox2, c-Myc, Klf4, Lin28, ASCL1, MYT1L, TBX3b, SV40 large T, hTERT, miR-291, miR-294, miR-295, and combinations thereof, and/or wherein the programming factor is selected from the group consisting of nerve growth factor (NGF), fibroblast growth factor (FGF), interleukin-6 (IL-6), bone morphogenic protein (BMP), neurogenin3 (Ngn3), pancreatic and duodenal homeobox 1 (Pdx1), Mafa, and combinations thereof.

6. The method of any one of claims 1 to 5, wherein the therapeutic protein is selected from the group consisting of adenosine deaminase (ADA), p91-PHOX, factor IX, factor VIII, cystic fibrosis transmembrane conductance regulator (CFTR), β-globin (HBB), dystrophin, hypoxanthine-guanine phosphoribosyltransferase (HGPRT), phenylalanine hydroxylase (PAH), glucosylceramidase (GBA and GBA2), fibrillin-1 (FBN1), huntingtin (HTT), apolipoprotein B (apoB), low density lipoprotein receptor (LDLR), low density lipoprotein receptor adaptor protein 1 (LDLRAP1), proprotein convertase subtilisin/kexin type 9 (PCSK9), synuclein alpha (SNCA), parkin (PRKN), leucine-rich repeat kinase 2 (LRRK2), PTEN induced putative kinase 1 (PINK1), parkinson protein 7 (DJ-1), ATPase type 13A2 (ATP13A2), cancer suicide gene products, anti-angiogenic factors, cancer self antigens including tyrosinase-related protein 2 (TRP-2) and carcinoembryonic antigen (CEA), immune stimulating factors, growth factors including granulocyte-macrophage colony-stimulating-factor (GM-CSF) and epithelial growth factor (EGF), cytokines including interleukins IL-2, IL-4, IL-5, IL-12, and IL-17, immunosuppressants, and combinations thereof.

7. The method of any one of claims 1 to 6 which is an *in vitro* method of reprogramming an at least partially differentiated cell to a less differentiated cell or programming a cell to be programmed to a desired differentiated state, the method comprising:
(a) providing an at least partially differentiated cell or a cell to be programmed from a donor,
(b) introducing at least one heterologous nucleic acid sequence into the cell provided in step (a) by infecting the cell with the recombinant negative-strand RNA virus vector comprising said at least one heterologous nucleic acid sequence, and
(c) culturing the infected cell under conditions effective to express said at least one heterologous nucleic acid sequence,
wherein said at least one heterologous nucleic acid sequence encodes a cellular reprogramming or programming factor.

8. The method of claim 7, wherein the at least partially differentiated cell is a mammalian cell selected from the group consisting of a terminally differentiated somatic cell, a precursor cell, a lineage-restricted stem cell, a somatic stem cell and a progenitor cell.

9. The method of claim 7 or 8, wherein the at least partially differentiated cell is reprogrammed to an oligopotent, multipotent or pluripotent cell.

10. The method of claim 7, wherein the cell to be programmed is a terminally differentiated somatic cell.

11. The method of any one of claims 1 to 6 which is an *in vivo* method of treating a genetic disorder, the method comprising
administering to a patient a recombinant negative-strand RNA virus vector comprising at least one heterologous nucleic acid sequence to introduce said at least one heterologous nucleic acid sequence into a cell of the patient,
wherein said at least one heterologous nucleic acid sequence encodes a therapeutic protein capable of treating said genetic disorder.

12. The method of any one of claims 1 to 6 which is an *in vitro* method of treating a genetic disorder, the method comprising:
(a) providing a cell from a donor,
(b) introducing at least one heterologous nucleic acid sequence into the cell provided in step (a) by infecting the cell with the recombinant negative-strand RNA virus vector comprising said at least one heterologous nucleic acid sequence, and
(c) culturing the infected cell under conditions effective to express said at least one heterologous nucleic acid sequence and/or to allow the cell to expand,
wherein said at least one heterologous nucleic acid sequence encodes a therapeutic protein capable of treating said genetic disorder.

13. A cell or a population of cells prepared *in vitro* by the methods of any one of claims 1 to 10 and 12.

14. The cell or a population of cells of claim 13 for use as a medicament.

15. A pharmaceutical composition comprising a cell or a population of cells according to claim 13, or a redifferentiated cell or a population of redifferentiated cells derived from a cell or a population of cells prepared by the *in vitro* method of reprogramming according to any one of claims 7 to 9.
